# EUROPÄISCHE PATENTSCHRIFT

(11) **EP 3 049 153 B1**
(45) Veröffentlichungstag und Bekanntmachung des Hinweises auf die Patenterteilung: **28.10.2020**
(21) Anmeldenummer: 14799656.5
(22) Anmeldetag: 23.09.2014
(51) Int. Cl.: C07K 7/64, C07K 14/47, A61K 38/12, G01N 33/68, A61P 25/28

(54) **ZYKLISCHE, AMYLOID-BETA-BINDENDE PEPTIDE UND DEREN VERWENDUNG**
CYCLIC AMYLOID-BETA-BINDING PEPTIDES AND THE USE THEREOF
PEPTIDES CYCLIQUES SE LIANT AUX BÊTA-AMYLOÏDES ET LEUR UTILISATION

(30) Priorität: 26.09.2013 DE 102013016002
(43) Veröffentlichungstag der Anmeldung: 03.08.2016
(73) Patentinhaber: Priavoid GmbH, 52428 Jülich (DE)
(72) Erfinder: WILLIBOLD, Dieter, 52428 Jülich (DE); BRENER, Oleksandr, 40591 Düsseldorf (DE)
(74) Vertreter: Fitzner, Uwe
(86) Internationale Anmeldenummer: PCT/DE2014/000476
(87) Internationale Veröffentlichungsnummer: WO 2015/043566

(56) Entgegenhaltungen:
- WO-A1-2013/021353
- WO-A1-2014/177127
- WO-A2-00/36093
- WO-A2-01/34631
- WO-A2-02/081505
- WO-A2-2013/150126
- WO-A2-2013/150127
- DE-A1-102012 102 998
- DE-A1-102012 102 999
- KAPURNIOTU APHRODITE ET AL: "Conformational restriction via cyclization in beta-amyloid peptide Abeta(1-28) leads to an inhibitor of Abeta(1-28) amyloidogenesis and cytotoxicity.", CHEMISTRY & BIOLOGY FEB 2003, Bd. 10, Nr. 2, Februar 2003 (2003-02), Seiten 149-159, XP026904712, ISSN: 1074-5521
- RAHIMIPOUR S ET AL: "In Vitro and Mechanistic Studies of an Anti-Amyloidogenic Self-Assembled Cyclic D,L-alpha-Peptide Architecture", BIOPOLYMERS, Bd. 100, Nr. 3, Sp. Iss. SI, Mai 2013 (2013-05), Seite 230, XP009182097, & 23RD AMERICAN PEPTIDE SYMPOSIUM; WAIKOLOA, HI, USA; JUNE 22 -27, 2013
- SIEVERS STUART A ET AL: "Structure-based design of non-natural amino-acid inhibitors of amyloid fibril formation", NATURE, NATURE PUBLISHING GROUP, UNITED KINGDOM, Bd. 475, Nr. 7354, 1. Juli 2011 (2011-07-01), Seiten 96-100, XP001526382, ISSN: 0028-0836, DOI: 10.1038/NATURE10154 in der Anmeldung erwähnt

### SEQUENCE LISTING

<110> Forschungszentrum Juelich GmbH
<120> zyklische, Amyloid-Beta-bindende Peptide und deren Verwendung
<130> PT 1.2634 PCT
<160> 11
<170> PatentIn version 3.5
<210> 1
   <211> 24
   <212> PRT
   <213> Artificial sequence
<220>
   <223> D-Peptid, cRD2D3
<400> 1
   Pro Thr Leu His Thr His Asn Arg Arg Arg Arg Arg Arg Pro Arg Thr
   1               5                   10                  15
   Arg Leu His Thr His Arg Asn Arg
               20
<210> 2
   <211> 24
   <212> PRT
   <213> Artificial Sequence
<220>
   <223> D-Peptide, cD3D3
<400> 2
   Arg Pro Arg Thr Arg Leu His Thr His Arg Asn Arg Arg Pro Arg Thr
   1               5                   10                  15
   Arg Leu His Thr His Arg Asn Arg
               20
<210> 3
   <211> 12
   <212> PRT
   <213> Artificial sequence
<220>
   <223> D-Peptide, cRD2
<400> 3
   Pro Thr Leu His Thr His Asn Arg Arg Arg Arg Arg
   1               5                   10
<210> 4
   <211> 24
   <212> PRT
   <213> Artificial Sequence
<220>
   <223> D-Peptide, cRD2RD2
<400> 4
   Pro Thr Leu His Thr His Asn Arg Arg Arg Arg Arg Pro Thr Leu His
   1               5                   10                  15
   Thr His Asn Arg Arg Arg Arg Arg
               20
<210> 5
   <211> 13
   <212> PRT
   <213> Artificial Sequence
<220>
   <223> D-Peptide, cD3r
<400> 5
   Arg Pro Arg Thr Arg Leu His Thr His Arg Asn Arg Arg
   1               5                   10
<210> 6
   <211> 13
   <212> PRT
   <213> Artificial Sequence
<220>
   <223> D-Peptide, cD3p
<400> 6
   Arg Pro Arg Thr Arg Leu His Thr His Arg Asn Arg Pro
   1               5                   10
<210> 7
   <211> 13
   <212> PRT
   <213> Artificial Sequence
<220>
   <223> D-Peptide, cD3a
<400> 7
   Arg Pro Arg Thr Arg Leu His Thr His Arg Asn Arg Ala
   1 5 10
<210> 8
   <211> 12
   <212> PRT
   <213> Artificial Sequence
<220>
   <223> D-Peptide, cD3
<400> 8
   Arg Pro Arg Thr Arg Leu His Thr His Arg Asn Arg
   1 5 10
<210> 9
   <211> 12
   <212> PRT
   <213> Artificial Sequence
<220>
   <223> D-Peptide, cDB3
<400> 9
   Arg Pro Ile Thr Arg Leu Arg Thr His Gln Asn Arg
   1 5 10
<210> 10
   <211> 24
   <212> PRT
   <213> Artificial Sequence
<220>
   <223> D-Peptide, cDB3DB3
<400> 10
   Arg Pro Ile Thr Arg Leu Arg Thr His Gln Asn Arg Arg Pro Ile Thr
   1 5 10 15
   Arg Leu Arg Thr His Gln Asn Arg
   20
<210> 11
   <211> 12
   <212> PRT
   <213> Artificial Sequence
<220>
   <223> D-Peptide, cD3(p2k)
<400> 11
   Arg Lys Arg Thr Arg Leu His Thr His Arg Asn Arg
   1               5                   10

## Beschreibung

Die Erfindung bezieht sich auf zyklische, Amyloid-Beta-bindende Peptide und deren Verwendung.

### Stand der Technik

Aus der DE 699 30 164 T2 ist die Intein-vermittelte Zyklisierung von Peptiden bekannt.

Aus DeMattos et al. (DeMattos, R.B., Bales, K.R., Cummins, D.J., Dodart, J.-C., Paul, S.M., Holtzman, D.M. (2001). Peripheral anti-Aß antibody alters CNS and plasma Aß dearance and decreases brain Aß burden in a mouse model of Alzheimer's disease. In: PNAS, Vol. 98, S. 8850-8855.) sind anti-Aß gerichtete Antikörper zur Behandlung der Alzheimerschen Demenz bekannt.

Es existiert aber weiterhin kein ursächlich wirkendes Medikament zur Behandlung der Alzheimerschen Demenz (AD, Alzheimersche Demenz, lat. Morbus Alzheimer). Eingesetzte Medikamente sind bestenfalls in der Lage, einige Symptome zu mildern, können aber den Krankheitsfortschritt nicht verlangsamen, geschweige denn aufhalten.

Es existiert eine Reihe von Substanzen, die in der Lage sind, im Tierversuch einige Erfolge bei der Prävention, nicht unbedingt aber bei der Behandlung der Alzheimerschen Demenz, zu erreichen.

Ein Merkmal der Alzheimer-Erkrankung sind extrazelluläre Ablagerungen des Amyloid-Beta-Peptids (A-Beta-Peptid, Aß oder Aß-Peptid). Diese Ablagerung des A-Beta-Peptids in Plaques ist typischerweise in den Gehirnen von AD-Patienten post mortem festzustellen. Deshalb werden verschiedene Formen des A-Beta-Peptids - wie z. B. Fibrillen - für die Entstehung und das Fortschreiten der Krankheiten verantwortlich gemacht. Zusätzlich werden seit einigen Jahren kleine, frei diffundierbare A-Beta-Oligomere als hauptsächliche Verursacher der Entstehung und des Fortschritts der AD gesehen. A-Beta-Monomere entstehen ständig in unserem Körper und sind vermutlich per se nicht toxisch. Es wird spekuliert, ob sich A-Beta-Mono-mere abhängig von ihrer Konzentration, die sich letztlich durch Bildungs- und Abbau-Raten im Körper ergibt, zufällig und damit mit zunehmendem Alter immer wahrscheinlicher spontan zu A-Beta-Oligomeren zusammen lagern. Einmal entstandene A-Beta-Oligomere könnten sich durch einen Prion-ähnlichen Mechanismus dann vermehren und letztlich zur Krankheit führen.

Derzeit existieren einige Substanzen, die auf verschiedenste Art und Weise die Konzentration von A-Beta-Monomeren verringern, beispielweise durch gamma-Sekretase-Inhibition oder -Modulation, A-Beta-bindende Antikörper, und so weiter, was offensichtlich ausreicht, um im Tierversuch (hier werden Tiere meist bereits behandelt, bevor die Krankheitssymptome voll ausbrechen) präventiv erfolgreich wirken. In klinischen Studien der Phasen II und III am Menschen dürfen nur klar mit der Alzheimerschen Demenz diagnostizierte Menschen behandelt werden. Hierin versagten bisher alle diese Substanzen. Möglicherweise, weil hier nach Beginn der Erkrankung eine geringe oder mäßige Verringerung der A-Beta-Monomer-Konzentration nicht mehr ausreicht, um die Entstehung immer größerer Mengen von A-Beta-Oligomeren zu verhindern.

Es gibt bisher keine Möglichkeit, die Alzheimersche Demenz vor dem Ausbruch der Symptome zu diagnostizieren. Die Alzheimersche Demenz wird heute hauptsächlich durch neuropsychiologische Tests der bereits an Demenzsymptomen leidenden Person erkannt. Des Weiteren können andere Erkrankungen, wie Traumata, durch verschiedene Untersuchungsmethoden ausgeschlossen werden. Es ist jedoch bekannt, dass A-Beta-Oligomere und zeitlich darauf folgend Plaques bis zu 20 Jahre vor dem Auftreten der Symptome im Gehirn der Patienten entstehen und irreversiblen Schaden anrichten. Molekulare Sonden, die dem Patienten intravenös injiziert werden und die nach der Passage über die Blut-Hirn-Schranke an A-Beta-Oligomere und Plaques binden, können mittels Bild gebender Methoden sichtbar gemacht werden und somit eine frühere Diagnose der Alzheimerschen Demenz ermöglichen.

Die existierenden A-Beta bindenden Substanzen sind nachteilig somit nicht genügend affin, um die Vermehrung von A-Beta-Oligomeren zu verhindern.

Es gibt nachteilig auch keine Sonden für das in-vivo-Imaging-Verfahren, die speziell an A-Beta-Spezies binden und diese sichtbar machen. Da A-Beta-Oligomere in der Krankheitsgeschichte eine so wichtige und frühe Rolle spielen, ist genau dieses wünschenswert.

### Aufgabe der Erfindung

Aufgabe der Erfindung ist es Peptide für eine
A) ursächliche Therapie von Morbus Alzheimer durch das Verhindern der Bildung von toxischen A-Beta-Oligomeren oder Aggregaten und/oder deren Enttoxifizierung, bzw. eine
B) sichere Diagnose der Alzheimerschen Demenz durch Bereitstellen von Sonden für ein in vivo-Imaging
   bereit zu stellen.

Im Nachfolgenden werden die Begriffe "A-Beta", "Amyloid-Beta", "Amyloid-β" bzw. "Aβ" synonym zueinander gebraucht.

### Lösung der Aufgabe

Die Aufgabe wird gelöst durch die erfindungsgemäßen Peptide nach dem Patentanspruch 1 und durch das Kit, durch die Zusammensetzung, durch die Sonde und den Verwendungen der Peptide gemäß den Nebenansprüchen. Vorteilhafte Ausgestaltungen hierzu ergeben sich jeweils aus den hierauf rückbezogenen Patentansprüchen.

### Beschreibung der Erfindung

Erfindungsgemäße Peptide umfassen mindestens ein an eine Amyloid-Beta-Spezies bindendes Peptid, wobei das Peptid eine lineare Aminosäuresequenz aufweist, die es dazu befähigen, an A-Beta zu binden, und diese Eigenschaft verstärkt wird, in dem das Peptid durch kovalente Verknüpfung in zyklisierter Form vorliegt, gekennzeichnet durch mindestens eine Struktur nach der Sequenz mit der SEQ ID NO:3 oder Homologe mit einer Identität von mindestens 83% zu SEQ ID NO:3 in zyklisierter Form.

Das Peptid D3 ist mit seiner lineraren Sequenz aus der Veröffentlichungsschrift WO 02/081505 A2 bekannt.

Die erfindungsgemäßen Peptide bestehen vorteilhaft im Wesentlichen aus D-enantiomere Aminosäuren. Im Sinne der vorliegenden Erfindung bedeutet der Begriff "im Wesentlichen aus D-enantiomere Aminosäuren", dass die einzusetzenden Aminosäuren mindestens 60 %, bevorzugt 75 %, 80 %, besonders bevorzugt 85 %, 90 %, 95 %, insbesondere 96 %, 97 %, 98 %, 99 %, 100 % aus D-enantiomere Aminosäuren aufgebaut sind.

In einer Ausgestaltung der Erfindung bindet das erfindungsgemäße zyklisierte Peptid Amyloid-Beta-Spezies mit einer Dissoziationskonstante (K_{D}-Wert) von höchstens 1 µM, bevorzugt 800, 600, 400, 200, 100, 10 nM, besonders bevorzugt 1000, 900, 800, 700, 600, 500, 400, 300, 200, 100 pM, insbesondere bevorzugt höchstens 50 pM, am meisten bevorzugt höchstens 25, 20, 15, 10, 9, 8, 7, 6, 5, 4, 3, 2, 1 pM bis sub-pM bindet, wobei jeder Zwischenwert angenommen werden kann.

Die Dissoziationskonstante (K_{D}-Wert) des zyklisierten Peptids ist dabei im Vergleich zu bindenden Peptiden mit linearer Sequenz, insbesondere aber im Vergleich zu vorzugsweise jedem der linearen Peptide, aus dem sich das zyklische Peptid ableiten lässt, vorteilhaft erniedrigt. Damit verbunden sind verbesserte Eigenschaften der erfindungsgemäßen Peptide, wie Affinität der Bindung und Effektivität des Abbaus und / oder der Verhinderung der Bildung toxischer Amyloid-Beta-Spezies. Dies betrifft insbesondere aber nicht ausschließlich einen niedrigeren K_{D}-Wert an der high affinity site des A-Beta (Monomer, Oligomer und Fibrillen).

Das erfindungsgemäße Peptid weist vorteilhaft eine Aminosäuresequenz auf, bei der die Zyklisierung des linearen Moleküls z. B. durch eine kovalente Bindung der ersten mit der letzten Aminosäure, z.B. über eine Kondensationsreaktion, erfolgt ist. Selbstverständlich existieren weitere Möglichkeiten der Zyklisierung, z. B. indem andere Aminosäuren miteinander verknüpft werden. Lediglich beispielhaft sei die Verknüpfung der zweiten Aminosäure mit der letzten Aminosäure genannt. Genauso denkbar ist jede mögliche andere Verknüpfung.

Im Falle, dass die erste und die letzte Aminosäure des Peptids miteinander verknüpft werden, wird vorteilhaft bewirkt, dass keine offenen Enden in der Peptidkette (Aminosäuresequenz) vorliegen.

Diese Maßnahme bewirkt ferner, dass alle Peptide mit linearen Aminosäuresequenzen, die nach der Zyklisierung die gleiche, nicht mehr unterscheidbare Aminosäure-Reihenfolge ergeben, in diesem Sinne identisch sind.

Beispiel: Die lineare Aminosäuresequenz des bekannten Peptids D3 ist rprtrlhthrnr. Das entsprechende durch eine Amidbindung zwischen der N-terminalen Aminogruppe und der C-terminalen Carboxylgruppe verknüpfte, zyklisierte Peptid "cD3" ist nicht mehr zu unterscheiden von den zyklisierten Peptiden prtrlhthrnrr, rtrlhthrnrrp, trlhthrnrrpr, rlhthrnrrprt, Ihthrnrrprtr, hthrnrrprtrl, thrnrrprtrlh, hrnrrprtrlht, rnrrprtrlhth, nrrprtrlhthr, oder rrprtrlhthrn.

Die Herstellung zyklisierter Peptide ist Stand der Technik, und kann beispielweise nach dem Verfahren wie es in DE 102005049537 A1 beschrieben ist, erfolgen.

Vorteilhaft bewirkt die Zyklisierung über die erste und letzte Aminosäure des Peptids, dass keine "offenen" Enden der Peptidkette mehr existieren, die oft Angriffspunkte für Peptid-abbauende Aktivitäten in Zellen, Tieren oder Menschen sind, z. B. durch Aminopeptidasen und Carboxypeptidasen.

Zyklisierte Peptide haben vorteilhaft eine größere Stabilität in Tier und Mensch als die entsprechenden linearen Peptide. Allerdings ist dieser Effekt allein für die vorliegende Erfindung nicht entscheidend, wie im Weiteren dargelegt wird. Er gilt im Übrigen, wie gezeigt wurde, auch nur für den Fall einer head-to-tail- oder tail-to-head-Zyklisierung, bei dem entsprechend beide Enden des linearen Peptids miteinander verknüpft werden.

Im Rahmen der Erfindung wurde vielmehr erkannt, dass die erfindungsgemäßen zyklisierten Peptide im Vergleich zu linearen, bindenden Peptiden, aus denen sich das zyklisierte Peptid ableiten lässt mit einer höheren Affinität an Abeta-Spezies binden, und zwar insbesondere auch an die besonders toxischen Amyloid-Beta-Oligomere. Das heißt, dass der K_{D}-Wert bei den zyklisierten Peptiden niedriger ist, als bei linearen Peptiden, und insbesondere niedriger ist, als bei linearen Peptiden, aus denen sie sich ableiten lassen.

In einer weiteren bevorzugten Ausgestaltung der Erfindung ist daher die Affinität der Bindung der erfindungsgemäßen zyklisierten Peptide im Vergleich zu linearen Peptiden, aus denen sie sich ableiten lassen, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 100%, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, insbesondere 200 %, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, insbesondere 300%, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, insbesondere 400%, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, vorteilhaft sogar 500%, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, besonders vorteilhaft 600%, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, besonders vorteilhaft 700%, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746, 747, 748, 749, 750, 751, 752, 753, 754, 755, 756, 757, 758, 759, 760, 761, 762, 763, 764, 765, 766, 767, 768, 769, 770, 771, 772, 773, 774, 775, 776, 777, 778, 779, 780, 781, 782, 783, 784, 785, 786, 787, 788, 789, 790, 791, 792, 793, 794, 795, 796, 797, 798, 799, ebenfalls besonders vorteilhaft 800%, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, ebenfalls besonders vorteilhaft 900%, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, oder sogar um 1000 %, oder sogar um 10000% oder sogar um bis zu 100000% oder 1000000% erhöht, wobei jeder Zwischenwert angenommen werden kann. Dies betrifft insbesondere aber nicht ausschließlich eine erhöhte Affinität zu der high affinity site des A-Beta (Monomer, Oligomer, Fibrillen und so weiter).

Dies wird durch einen entsprechend erniedrigten K_{D}-Wert angezeigt. Der K_{D}-Wert als Maß für die Affinität der Bindung des zyklisierten Peptids an Amyloid-Beta-Spezies und insbesondere an Amyloid-Beta-Oligomer ist im Vergleich zu einem linearen, bindenden Peptid, vorzugsweise gegenüber jedem linearen, bindenden Peptid, aus denen sich das zyklisierte Peptid ableiten lässt, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 99,1, 99,2, 99,3, 99,4, 99,5%, 99,6, 99,7, 99,8, 99,9 bis zu 99,99 oder sogar 99,999% erniedrigt.

Diese niedrigeren K_{D}-Werte beziehen sich vorteilhaft insbesondere aber nicht ausschließlich auf die high affinity site des A-Beta (Monomer, Oligomer, Fibrillen und so weiter).

Erfindungsgemäße zyklisierte Peptide können daher effizienter als Sonden für diagnostische Zwecke verwendet werden, als lineare, bindende Peptide, insbesondere auch effizienter als ihre linearen Peptid-Pendants, aus denen sie sich ableiten lassen (gleiche Aminosäuresequenz), verwendet werden.

Sie können aber insbesondere auch als Therapeutika effizienter als lineare Peptide, insbesondere effizienter als ihre linearen Peptid-Pendants, aus denen sie sich ableiten lassen, verwendet werden.

Im Rahmen der Erfindung wurde nämlich weiterhin erkannt, dass die erfindungsgemäßen zyklisierten Peptide im Vergleich zu linearen Peptiden, insbesondere aber zu ihren Peptid-Pendants mit linearen Sequenzen, aus denen sie sich ableiten lassen, zusätzlich auch mit einer höheren Effektivität bzw. Effizienz die Bildung besonders toxischer Amyloid-Beta-Oligomere verhindern oder deren Zerstörung und / oder Endtoxifizierung bewirken. Diese Effektivität ist insbesondere um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99,9, besonders vorteilhaft sogar um 100% erhöht.

Eine Probe mit den verschiedenen Aß-Konformeren wird hierzu testweise im einfachsten Fall z. B. fraktioniert. In jeder Fraktion werden entsprechend des Fraktionierungsschritts verschiedene Konformere, wie Monomere, Oligomere, Fibrillen oder höhrere Aggregate angereichert und können sodann exakt bestimmt werden.

Der Begriff "exakt bestimmt" umfasst einen Kalibrierungsschritt bei der Fraktionierung durch Moleküle bekannter Art und Verhaltens. Nach der Fraktionierung liegt in jeder Fraktion nur eine bestimmte Art an Konformeren des Aß vor, z. B. Monomere, Oligomere oder Fibrillen und so weiter.

Beispielweise werden die Konformere bei einer Dichtegradientenzentrifugation als Fraktionierungsschritt nach ihrem s-Wert oder Sedimentationskoeffizienten aufgetrennt. Moleküle unterschiedlicher Größe können einen identischen hydrodynamischen Radius besitzen, haben aber dennoch unterschiedliche s-Werte und werden danach auch aufgetrennt. Durch eine Kalibration mit Molekülen von bekanntem s-Wert sind die mittels Dichtegradientenzentrifugation erhaltenen Aß Konformere exakt nach ihrem s-Wert bestimmt.

Im Weiteren werden die erhaltenen Fraktionen mit und ohne Wirkstoff behandelt und z. B. durch RP-HPLC bestimmt. Auf diese Weise kann man die Effektivität des Wirkstoffs bestimmen.

Ein weiteres Verfahren wird nachfolgend vorgestellt. Zur Quantitativen Analyse von Wirkstoffen kann der sogenannte QIAD-Test eingesetzt (quantitative determination of interference with Aß aggregate size distribution). Das Verfahren zur quantitativen Analyse des Einflusses eines Wirkstoffs auf die Partikelgrößenverteilung amyloider Peptide und / oder Proteine in einer Probe, weist dabei nachfolgende Schritte auf. Zunächst lässt man A-Beta unter kontrollierten Bedingungen aggregieren, so dass je verschiedene A-Beta-Aggregate entstehen. Die Bedingungen werden so gewählt, dass besonders viele, kleine, besonders cytotoxische A-Beta-Oligomere gebildet wurden. Als nächstes wird die zu untersuchende Substanz, z.B. eines der erfindungsgemäßen zyklisierten Peptide zu der Probe zugegeben. Der Wirkstoff ändert die Partikelgrößenverteilung in der Probe. Diese Änderung wird quantitativ festgestellt. Die Änderung ist ein Maß für die Reduktion oder sogar für die vollständige Eliminierung bestimmter toxischer Spezies einer bestimmten Partikelgröße. Durch das QIAD-Verfahren wird die Zunahme oder die Abnahme von A-Beta-Aggregaten einer bestimmten Partikelgröße gemessen. Während einige A-Beta-Aggregate mit einer bestimmten Größe anfänglich in der Probe vorhanden waren, werden diese unter Einfluss des Wirkstoffs reduziert oder sogar vollständig eliminiert. Andere Partikelgrößen nehmen unter dem Einfluss des Wirkstoffs zu oder bleiben konstant. Die Partikel, die aus dem A-Beta gebildet sind, werden vorzugsweise nach ihrem hydrodynamischen Radius der Partikel voneinander getrennt. Auf diese Weise wird vorteilhaft bewirkt, dass aus der Probe eine Mehrzahl an Fraktionen erhalten wird. In den Fraktionen sind die Partikel an amyloiden Peptiden und / oder Proteinen mit einer bestimmten Aggregatgröße angereichert. Diese Trennung der Partikel kann mittels einer Dichtegradientenzentrifugation vorgenommen werden. Die Fraktionen werden räumlich voneinander getrennt, z. B. durch Abpipettieren. Abschließend wird die Konzentration an A-Beta in der jeweiligen Fraktion durch eine vollständige Denaturierung der A-Beta-Spezies während einer im Anschluss an die Fraktionierung durchgeführten Umkehrphasen (RP-) HPLC bestimmt. Die Denaturierung der Aggregate kann z. B. mit 30 % Acetonitril und 0,1 % Trifluoressigsäure bei einer Säulentemperatur von 80 °C vollständig erfolgen und auf einer C8-Säule nach Hydrophobizität aufgetrennt. Eluierendes A-Beta wird mittels der UV-Absorption bei 215 nm detektiert. Die Peakflächenintegration kann mittels Agilent Chemstation Software erfolgen. Die Verrechnung daraus entstandener Werte mit einer zuvor durchgeführten Kalibrierung erlaubt die Berechnung der Konzentration des in der jeweiligen Fraktion vorhandenen A-Beta. Je Fraktion kann der Mittelwert aus mehreren z. B. sechs voneinander unabhängig durchgeführten Experimenten mit der sich ergebenen Standardabweichung berechnet werden. Der Vorteil der HPLC-Analyse liegt darin, dass man unabhängig vom Aggregationszustand und eines Lösungsmittels sehr sensitiv detektieren (z. B. ca. 20 nM oder 1,8 ng Aß1-42) und zuverlässig quantifizieren kann. Ein entscheidender Vorteil des Verfahrens besteht in der Kopplung von Dichtegradientenzentrifugation und Umkehrphasen HPLC, welche eine zuverlässige Quantifizierung auch von Aß-Oligomeren möglich macht.

Der erfindungsgemäße Effekt einer gesteigerten Effektivität der Eliminierung (bzw. der Bildung) von Amyloid-Beta-Spezies und insbesondere Amyloid-Beta-Oligomeren kann mit einem dieser Verfahren, aber nicht ausschließlich mit diesen Verfahren erfolgen.

In einer besonders bevorzugten Ausgestaltung der Erfindung treten die genannten Effekte der gesteigerten Affinität und Effektivität der Eliminierung, Endtoxifizierung (bzw. der Bildung) sogar *in vitro* und / oder *in vivo* auf.

Das zyklisierte Peptid kann eine Linker-Gruppe aufweisen. Mit dem Begriff Linker-Gruppe sind vorzugsweise gemeint: zusätzliche einzelne Aminosäuren in Molekülen wie z. B. D3, die per se bei der Behandlung der Alzheimerschen Demenz eingesetzt werden können, um A-Beta-Bestandteile zu binden. Durch die zusätzliche Aminosäure soll die Bindung des zyklisierten Peptids an A-Beta nicht beeinträchtigt werden.

Einige beispielhafte Sequenzen zu zyklischen Peptiden, die offenbarungsgemäß einsetzbar sind, sind nachfolgend dargestellt:
cD3: rprtrlhthrnr (SEQ ID NO: 8; nicht erfindungsgemäß),
cRD2: ptlhthnrrrrr (SEQ ID NO: 3; erfindungsgemäß) oder Vielfache und Homologe hiervon.

Es sind alle anderen wirksam an A-Beta bindende lineare Peptide in zyklisierter Form vorteilhaft offenbarungsgemäß umfasst.

In einer besonders vorteilhaften Ausgestaltung der Erfindung weist das erfindungsgemäße Peptid mindestens 2, 3, 4, 5, 6, 7, 8, 9, 10 oder mehr jeweils für sich getrennt wirksam an A-Beta bindende Peptid-Bausteine auf, wobei es sich dabei um identische oder um nicht-identische Peptid-Bausteine handeln kann. Diese Peptid-Bausteine sind Kopf an Kopf, Schwanz an Schwanz oder Kopf an Schwanz linear gekoppelt und insgesamt durch kovalente Verknüpfung, mit oder ohne zusätzliche Linker-Bausteine (z. B. ein oder mehrere Aminosäuren) zyklisiert, z. B. über ihren beiden Enden.

Die mindestens zwei Peptid-Monomer-Einheiten, können dabei wiederum kovalent oder nicht-kovalent, z. B. über eine Biotin-Gruppe und einen Streptavidin-Tetramer miteinander verknüpft sein. Die Peptide sind durch Einheiten gekennzeichnet, welche Kopf an Kopf, Schwanz an Schwanz oder Kopf an Schwanz linear gekoppelt sind.

Lediglich beispielhaft seien genannt:
cRD2D3: ptlhthnrrrrrrprtrlhthrnr (SEQ ID NO: 1)*
cD3D3: rprtrIhthrnrrprtrIhthrnr (SEQ ID NO: 2)*
cRD2: ptlhthnrrrrr (SEQ ID NO: 3)
cRD2RD2, ptlhthnrrrrrptlhthnrrrrr (SEQ ID NO: 4)*
cD3r, rprtrlhthrnrr (SEQ ID NO: 5)*
cD3p, rprtrlhthrnrp (SEQ ID NO: 6)*
cD3a, rprtrlhthrnra (SEQ ID NO: 7)*
cD3: rprtrlhthrnr (SEQ ID NO: 8)*
cDB3, rpitrlrthqnr (SEQ ID NO: 9)*
cDB3DB3, rpitrlrthqnrrpitrlrthqnr (SEQ ID NO: 10)*
cD3(p2k), rkrtrlhthrnr (SEQ ID NO: 11)*
oder Vielfache und Homologe hiervon. (* = nicht erfindungsgemäß)

Es kann auch jede beliebige Kombination aus 2, 3, 4, 5, 6, 7, 8, 9, 10 oder mehr der oben beschriebenen Sequenzen in zyklisierter Form das offenbarungsgemäße Peptid ausbilden.

Da das Zielmolekül der therapeutischen Behandlung vorzugsweise ein A-Beta-Oligomer und damit natürlicherweise ein multivalentes Target ist, werden in einer besonders bevorzugten Ausgestaltung der Erfindung die für eine Behandlung, das heißt Vernichtung vorhandener A-Beta-Oligomere Substanz eingesetzt, die aus mehreren Kopien einer bereits effizient A-Beta-Oligomer-bindenden Einheit oder aus mehreren verschiedenen bereits effizient A-Beta-Oligomer-bindenden Einheiten besteht. Diese Peptide sind ebenfalls zyklisiert. In diesem Fall umfasst das erfindungsgemäße Peptid also mehrere, an sich wirksam an A-Beta-Spezies und insbesondere -Oligomer bindende Peptid-Bausteine, welche jeweils aus Aminosäuren aufgebaut und insgesamt durch kovalente Verknüpfung zyklisiert ist.

In einer weiteren Variante weisen die Peptid-Bausteine Fragmente der oben genannten Sequenzen auf oder weisen homologe Sequenzen zu den oben genannten Sequenzen auf.

"Homologe Sequenzen" oder "Homologe" bedeutet im Sinne der Erfindung, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung gleich, wenn sie die gleiche Aminosäuresequenz besitzen.

Insbesondere sind alle linearen Peptide mit Aminosäuresequenzen, die nach der Zyklisierung die gleiche, nicht mehr unterscheidbare Aminosäure-Reihenfolge ergeben, in diesem Sinne identisch. Beispiel: Die lineare Aminosäuresequenz von D3 ist rprtrlhthrnr. Das entsprechende durch eine Amidbindung zwischen der N-terminalen Aminogruppe und der C-terminalen Carboxylgruppe verknüpfte, zyklisierte Peptid "cD3" ist nicht mehr zu unterscheiden von den zyklisierten Peptiden prtrlhthrnrr, rtrlhthrnrrp, trlhthrnrrpr, rlhthrnrrprt, Ihthrnrrprtr, hthrnrrprtrl, thrnrrprtrlh, hrnrrprtrlht, rnrrprtrlhth, nrrprtrlhthr, oder rrprtrlhthrn. Die positiven Effekte der offenbarungsgemäßen zyklisierten Peptide in Hinblick auf Affinität und Effektivität treten darüber hinaus gegenüber mindestens einem, vorzugsweise gegenüber jedem linearen bindenden Peptid, aus denen es sich ableiten lässt, auf. Also im Falle von zyklisierten D3 gegenüber linearen bindenden Peptiden mit der Sequenz prtrlhthrnrr, rtrlhthrnrrp, trlhthrnrrpr, rlhthrnrrprt, Ihthrnrrprtr, hthrnrrprtrl, thrnrrprtrlh, hrnrrprtrlht, rnrrprtrlhth, nrrprtrlhthr, oder rrprtrlhthrn. Offenbarungsgemäße zyklisierte Peptide zeigen in diesem Zusammenhang a) eine höhere Bindungsaffinität an Amyloid-Beta-Spezies als mindestens ein, vorzugsweise jedes der linearen Peptide, aus denen es sich ableiten lässt. Offenbarungsgemäße zyklisierte Peptide zeigen b) eine höhere Effektivität bei der Verhinderung der Bildung und / oder der Endtoxifizierung insbesondere von Amyloid-Beta-Oligomer, als mindestens ein, vorzugsweise jedes der linearen, bindenden Peptide, aus denen sich das zyklisierte Peptid ableiten lässt. Das oben genannte zyklisierte D3 ist dabei lediglich beispielhaft zu verstehen. Die Effekte treten also insbesondere auch aber nicht ausschließlich bei den zyklisierten Peptiden mit der SEQ ID NO: 1-11 auf.

Unter Homologe sind in einer Variante die entsprechenden retro-inversen Sequenzen der oben genannten Bausteine zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

Die erfindungsgemäßen Peptid-Bausteine und Peptid-Polymere, im Allgemeinen gesprochen: die erfindungsgemäßen Peptide, sind geeignet zur Verwendung in der Medizin.

In einer Ausführung handelt es sich um erfindungsgemäße Peptide zur Behandlung von Morbus Alzheimer. In einer weiteren Ausführung handelt es sich um erfindungsgemäße Peptide, welche zur Behandlung von Parkinson, CJD (Creutzfeldt Jakob Disease), ALS (Amyotrophe Lateralsklerose) oder andere neurodegenerative Erkrankungen, oder Diabetes eingesetzt werden kann.

Weiterer Gegenstand der vorliegenden Erfindung ist ein Kit, enthaltend das erfindungsgemäße Peptid. In einem solchen Kit können die erfindungsgemäßen Peptide in Behältern ggf. mit/in Puffern oder Lösungen verpackt sein. Alle Komponenten des Kits können in demselben Behälter oder getrennt voneinander verpackt sein. Ferner kann das Kit Anweisungen für dessen Gebrauch enthalten. Ein solches Kit kann beispielsweise die erfindungsgemäßen Peptide in einer Injektionsflasche mit Stopfen und/oder Septum enthalten. Ferner kann darin beispielsweise auch eine EinmalSpritze enthalten sein.

Die vorliegende Erfindung betrifft auch die Verwendung der erfindungsgemäßen Peptide als Sonde zur Identifizierung, qualitativen und/oder quantitativen Bestimmung von insbesondere Amyloid-Beta-Oligomeren. Gegenstand der vorliegenden Erfindung ist auch die Sonde als solche, enthaltend das erfindungsgemäße Peptid zur Identifizierung, qualitativen und/oder quantitativen Bestimmung von insbesondere Amyloid-Beta-Oligomeren.

Solche Sonden sind von großer Bedeutung, da damit eine frühe Diagnose der Alzheimerschen Demenz möglich wird. Damit kann der Krankheit schon in einem sehr frühen Stadium entgegengewirkt werden. Ferner kann so der Fortschritt der Erkrankung oder eines Therapie-Versuches oder einer Therapie verfolgt werden.

Solche molekularen Sonden enthalten das erfindungsgemäße Peptid und können den Patienten z. B. intravenös injiziert werden. Weitere Bestandteile der Sonden können sein: Farbstoffe, Fluoreszenzfarbstoffe, radioaktive Isotope (z. B. für PET), Gadolinium (für MRI) und/oder Bestandteile, die für Sonden in der Bildgebung eingesetzt werden. Vor oder nach der Passage über die Bluthirnschranke können die Sonden insbesondere an A-Beta-Oligomere und/oder Plaques binden. Die so markierten Amyloid-Beta-Spezies und insbesondere die A-Beta-Oligomere und/oder Plaques können mittels bildgebender Verfahren wie z. B. SPECT, PET, CT, MRT, NIR (near infrared), Protonen-MR-Spektroskopie und so weiter sichtbar gemacht werden.

Weiterer Gegenstand der vorliegenden Erfindung ist die Verwendung des zyklisierten Peptids zur Verhinderung der Bildung und/oder der Endtoxifizierung von toxischen Amyloid-Beta-Oligomeren. Die erfindungsgemäßen Peptide werden also auch zur Bildung von nicht-toxischen Peptid-Amyloid-Beta-Oligomer-Komplexen eingesetzt.

Gegenstand der vorliegenden Erfindung ist auch eine Zusammensetzung enthaltend das erfindungsgemäße Peptid, insbesondere zur Behandlung von Morbus Alzheimer.

Gegenstand der vorliegenden Offenbarung ist ferner eine Zusammensetzung enthaltend das erfindungsgemäße Peptid, insbesondere zur Verhinderung von toxischen A-Beta-Oligomeren oder zur Zerstörung von daraus gebildeten Polymere oder Fibrillen.

Die erfindungsgemäße "Zusammensetzung" kann z. B. ein Impfstoff, ein Medikament (z. B. in Tablettenform), eine Injektionslösung, ein Nahrungs- oder Nahrungsergänzungsmittel sein enthaltend das erfindungsgemäße Peptid in einer aufgrund des Fachwissens herzustellenden Formulierung.

Die erfindungsgemäßen Peptide enttoxifizieren die A-Beta-Oligomere oder daraus gebildete Aggregate sowie Fibrillen, indem sie daran binden und so in nicht toxische Verbindungen überführen. Substanzen, die in der Lage sind, die Bildung von Fibrillen zu inhibieren, müssen nicht notwendigerweise auch in der Lage sein, vorgeformte Fibrillen zu zerstören, da vorgeformte A-Beta-Fibrillen sehr stabil sind und nur sehr schwer und langsam wieder zerstört werden können.

Demgemäß ist Gegenstand der vorliegenden Offenbarung auch ein Verfahren zur Enttoxifizierung der A-Beta-Oligomeren, daraus gebildeten Polymeren oder Fibrillen.

Es wurde erkannt, dass wenn bereits Aß-Oligomere vorhanden sind, es das Ziel einer Behandlung sein muss, diese durch Substanzen zu adressieren, die eine möglichst hohe Affinität zu A-Beta besitzen. De facto kann die Affinität gar nicht groß genug sein und die entsprechende Dissoziationskonstante des erfindungsgemäßen Peptids liegt dann im pM-Bereich oder noch tiefer.

Es wurde ferner erkannt, dass ein wichtiger Unterschied zwischen Prävention und Behandlung in nachfolgender Überlegung liegt. Um die Bildung der ersten A-Beta-Oligomere zu verhindern, reichen eventuell schon wenig affine und effektive A-Beta-Liganden aus. Da die Bildung eines A-Beta-Oligomers aus mehreren A-Beta-Monomeren eine Reaktion sehr hoher Ordnung ist, ist sie in hoher Potenz von der A-Beta-Monomer-Konzentration abhängig. Auch eine kleine Verringerung der aktiven A-Beta-Monomer-Konzentration kann so die Bildung der ersten A-Beta-Oligomere verhindern. Dies ist die Situation bei der Prävention.

Sind aber bereits A-Beta-Oligomere entstanden, können sich diese (Prion-ähnlich) vermehren, was keine Reaktion hoher Ordnung ist und damit kaum noch abhängig von der A-Beta-Monomer-Konzentration ist. Dies ist die Situation bei der Therapie. Sind also A-Beta-Oligomere bereits entstanden, muss es das Ziel einer Behandlung sein, diese durch Substanzen zu adressieren, die eine möglichst hohe Affinität zu Aß-Oligomeren besitzen und / oder diese besonders effizient eliminieren und / oder der Bildung besonders effizient verhindern. Die entsprechende Dissoziationskonstante müsste dazu im sub-µM, nM oder pM-Bereich oder noch tiefer liegen. Dies ist in der vorliegenden Erfindung der Fall, und die erfindungsgemäßen zyklisierten Peptide binden im Sinne der Therapie die Amyloid-Beta-Spezies und insbesondere die A-Beta-Oligomere mit entsprechend niedriger Dissoziationskonstante.

Aufgrund ihrer Eigenschaft sowohl die kleinen, frei diffundierbaren A-Beta-Oligomere, größere A-Beta-Oligomere bis hin zu Fibrillen zu binden, sind die erfindungsgemäßen Peptide in allen Stadien der Alzheimerschen Demenz einsetzbar. Auf Basis der Lehre der vorliegenden Erfindung können Peptide hergestellt werden, die auch selektiv an verschiedene Formen der A-Beta-Oligomere binden.

Neben den bereits genannten Verfahren zur Herstellung der erfindungsgemäßen Peptide sind beispielsweise Peptid-Synthese-Verfahren, die rekombinante Herstellung von Proteinen und anerkannte organische Synthese-Methoden für beliebige sogenannte low-molecular-weight compounds möglich.

Denkbar ist insbesondere eine kovalente Zyklisierung dieses linearen Moleküls. Dadurch werden insbesondere ein vorteilhaft sogar beide offenen Enden der Peptidkette vermieden. Dies erhöht einerseits die Stabilität des Peptides in Tier und Mensch, was zu deutlich vorteilhafteren pharmakokinetischen Eigenschaften führt. Durch die Zyklisierung wird aber auch besonders vorteilhaft bewirkt, dass die Zahl an möglichen Konformationen des Peptides im ungebundenen Zustand stark reduziert wird. Dies wiederum reduziert die Entropie im freien Zustand, was wieder den entropischen Teil der Bindungsreaktion zum Vorteil der Komplexbildung mit dem Zielmolekül Abeta vorteilhafter macht. Dadurch wird letztlich die Affinität zum Zielmolekül im Vergleich zum linearen, bindenden Peptid, aus denen sich das zyklisierte Peptid ableiten lässt, deutlich erhöht. Gleiches gilt für die Effektiviät der Eliminierung und / oder der Verhinderung der Bildung toxischer Amyloid-Beta-Spezies und insbesondere der Amyloid-Beta-Oligomere. Diese erfindungsgemäßen Effekte sind dabei entscheidend, und nicht so sehr eine möglicherweise gesteigerte Stabilität, die lediglich ein positiver Nebeneffekt der Zyklisierung ist.

Diese Zyklisierung erfolgt z. B. durch head-to-tail (Kopf zu Schwanz) oder tail-to-head (Schwanz an Kopf) oder andere Zyklisierungen des linearen Peptids. Jede mögliche Zyklisierung kann hierbei in Betracht gezogen werden.

Da das Zielmolekül der therapeutischen Behandlung insbesondere ein A-Beta-Oligomer und damit natürlicherweise ein multivalentes Target ist, ist die Idee, die für eine Behandlung (Vernichtung vorhandener A-Beta-Oligomere) einzusetzende Substanz aus mehreren Kopien einer bereits effizient A-Beta-Oligomer-bindenden Peptid-Einheit herzustellen, die vorteilhafterweise zusätzlich zyklisiert sind. In diesem Fall umfasst das Peptid mehrere, als solche bereits wirksam an A-Beta-Oligomer bindende Peptid-Bausteine, welche jeweils linear aus Aminosäuren aufgebaut und insgesamt zwischen seinen verbleibenden beiden Enden kovalent zu einem Zyklus verbunden sind.

Durch diese Maßnahme wird vorteilhaft eine weitere Erniedrigung der KD bewirkt. Es wird also vorteilhaft bewirkt, dass, zumindest theoretisch und bei Abwesenheit störender Einflüsse, z. B. durch sterische Behinderung, ein n-mer einer A-Beta-Oligomer-bindenden Einheit, welche an A-Beta-Oligomere mit einer Dissoziationskonstante (KD) von x bindet, eine apparente KD von x hoch n erreicht.

Dabei existieren vielfältige Möglichkeiten, dies zu erreichen: Die A-Beta-Oligomer-bindenden Einheiten, das heißt die Peptid-Monomer-Einheiten, können kovalent oder nicht-kovalent, z. B. über eine Biotin-Gruppe und einen Streptavdin-Tetramer miteinander verknüpft werden.

Ein kovalente Verknüpfung kann erreicht werden, in dem die Monomer-Einheiten Kopf an Kopf, Schwanz an Schwanz oder Kopf an Schwanz linear gekoppelt werden, und zwar ohne eine Linker-Gruppe oder mit einer Linker-Gruppe. Dabei können auch nicht-identische Momomer-Einheiten kombiniert und erfindungsgemäß zyklisiert werden.

### Ausführungsbeispiele

Im Weiteren wird die Erfindung an Hand von Ausführungsbeispielen und der beigefügten Figuren näher erläutert. Hierdurch wird keinerlei Beschränkung der Erfindung vorgenommen. Vielmehr ist dem Fachmann klar, dass er im Rahmen seines Fachwissens andere Peptide, die nachgewiesenermaßen wirksam an A-Beta-Spezies binden, in zyklisierter Form herstellen und in der Prävention und Behandlung der Alzheimerschen Demenz einsetzen kann.

Es zeigen:
- Figur 1: Veränderung der Fraktionen einer standardisierten Dichtegradientenzentrifugation nach Zugabe von linearem D3 (Stand der Technik) und cD3 (zyklisiertes D3). Der Vergleich des Einflusses von D3 (schraffierte Balken) und cD3 (schwarz) auf die Größenverteilung der A-BetaAggregate (Kontrolle in weiß), zeigt, dass cD3 die Oligomere in Fraktionen 4 bis 8 noch effizienter eliminiert als lineares D3.
- Figur 2:: K_{D}-Werte (Kinetik) zu Amyloid-Beta-Monomer
- Figur 3:: K_{D}-Werte (Kinetik) zu Amyloid-Beta-Oligomer
- Figur 4:: K_{D}-Werte (Kinetik) zu Amyloid-Beta-Fibrillen
- Figur 5:: Moris-Water-Maze Test (MWW)

Das D-enantiomere lineare Peptid mit dem Namen D3 ist aus der Veröffentlichung WO 02081505 A2 bekannt. Es wurde durch eine Spiegelbild-Phagendisplay-Selektion gegen überwiegend monomeres A-Beta (1-42) identifiziert, mit dem Plan, dieses durch die Bindung zu stabilisieren und seine Umwandlung in toxische A-Beta-Aggregate zu verhindern. Nach derzeitigem Kenntnisstand bindet D3 bevorzugt die besonders toxischen A-Beta-Oligomere, präzipitiert diese und wandelt sie dabei in nicht-toxische, nicht-amyloidogene und ThT-negative amorphe Aggregate um. Im Tiermodell wird selbst durch eine orale Verabreichung von D3 mit dem Trinkwasser erreicht, dass behandelte transgene AD-Mäuse deutlich weniger Plaques enthalten und signifikant verbesserte kognitive Fähigkeiten besitzen.

### Erstes Ausführungsbeispiel:

Das erste Ausführungsbeispiel bezieht sich darauf, die Steigerung der Effektivität bzw. Effizienz von D3 beim Abbau der Amyloid-Beta-Oligomere durch Zyklisierung zu zeigen. Hierzu wurde das N-Termini und das C-Termini der ersten und der letzten Aminosäure des linearen bindenden Peptids D3 durch eine Peptidbindung kovalent verbunden.

Zyklisiertes D3 ("cD3"; nicht erfindungsgemäß) wurde von der Firma peptides&elephants GmbH (Am Mühlenberg 11, 14476 Potsdam-Golm, Deutschland) erhalten.

Zur Herstellung der Fratkionen aus der Dichtegradientenzentrifugation wurde nachfolgendes Verfahren angewendet. Die Konformere wurden nach ihrem s-Wert oder Sedimentationskoeffizienten aufgetrennt. Moleküle unterschiedlicher Größe können einen identischen hydrodynamischen Radius besitzen, haben aber dennoch unterschiedliche s-Werte und werden danach auch aufgetrennt. Durch eine Kalibration mit Molekülen von bekanntem s-Wert sind die mittels Dichtegradientenzentrifugation erhaltenen A-Beta-Konformere exakt nach ihrem s-Wert bestimmt. Die Dichtegradientenzentrifugation weist dabei den Vorteil auf, dass hiermit alle A-Beta-Konformere in einem einzigen Verfahrensschritt voneinander getrennt werden können. Durch den Einsatz eines Dichtegradientenzentrifugationsschritts vor der Immobilisierung, bei dem auf einen vorgeformten Dichtegradienten Oligomere oder höhere A-Beta-Formen aufgeschichtet und die darin enthaltenen Aggregat-Partikel entsprechend ihres s-Werts durch Ultrazentrifugation getrennt werden, lassen sich im Laufe dieser Zentrifugation unterschiedliche A-Beta-Aggregate (Oligomere und Fibrillen oder amorphe Aggregate) nach ihrem unter anderem von der Partikelgröße abhängigen Sedimentationskoeffizienten voneinander trennen und fraktionieren. Die Fraktion mit dem gewünschten A-Beta-Konformer kann anschließend direkt auf eine Streptavidin beladene Sensoroberfläche zur Immobilisierung injiziert werden. Die Vorbereitung des Dichtegradienten erfolgte durch nachfolgendes Überschichten der Dichtegradientenlösung (lodixanol verdünnt in 10 mM Natriumphosphatpuffer pH 7,4) in Konzentrationen von 50 % (260 µl), 40 % (260 µl), 30 % (260 µl), 20 % (260 µl), 10 % (260 µl), und 5 % (100 µl) (v/v). Im Anschluss wurden die Proben auf den Dichtegradienten pipettiert und die Zentrifugation für 3 Stunden bei 4 °C und 259000 g durchgeführt. Nun wurden von dem Dichtegradienten insgesamt 14 Fraktionen zu je 140 µl von oben nach unten entnommen. Monomere sind in den ersten Fraktionen zu finden, Oligomere insbesondere ab den Fraktionen 4 und Fibrillen befinden sich typischerweise in den Fraktionen 11 bis 13.

Die erhaltenen Fraktionen aus der Dichtegradientenzentrifugation wurden entweder unbehandelt (Kontrolle: jeweils linke, nicht ausgefüllte Säule) oder mit dem aus dem Stand der Technik bekannten D3, (D3, 20 µM: jeweils mittlere, schraffierte Säule) oder aber mit der zyklisierten Variante des Moleküls D3, bei der das N-Termini und das C-Termini der ersten und der letzten Aminosäure durch eine Peptidbindung kovalent verbunden wurden (cD3, 20 µM: jeweils rechte, schwarze Säule), behandelt.

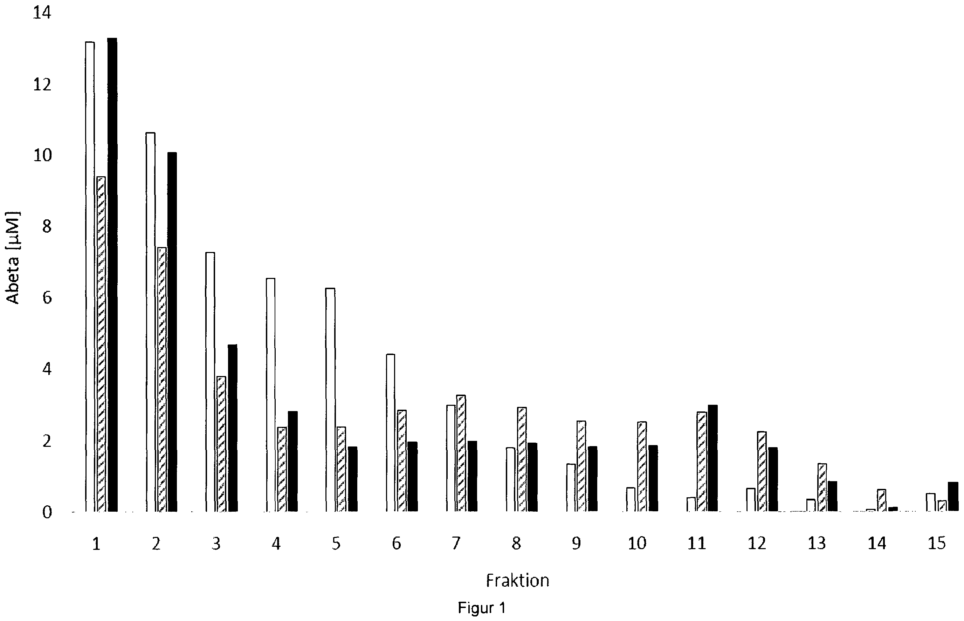

Das Ergebnis ist in der Figur 1 dargestellt. Es wird deutlich, dass das zyklisierte cD3 vorteilhaft keinen Einfluss auf die A-Beta-Monomer-Konzentration der Fraktionen 1-2 zeigt.

In Hinblick auf die Fraktionen 4-8 bzw. 4-10 ist aber vorteilhaft eine deutliche Abnahme der A-Beta-Oligomer-Konzentration erkennbar.

Ein vergleichbarer Effekt tritt auch bei den erfindungsgemäßen Peptiden auf.

### Zweites Ausführungsbeispiel:

Die nachfolgenden Schritte beziehen sich sowohl auf Affinitätsstudien als auch auf Studien zum Abbau besonders toxischer Amyloid-Beta-Oligomere.

### Herstellung von Aß-Monomeren, Oligomeren und Fibrillen

1 mg lyophilisiertes Aß1-42 und N-terminal biotinyliertes Aß1-42 wurden jeweils in 1 ml 100% Hexafluorisopropanol (HFIP) gelöst und bei Raumtemperatur über Nacht gelöst. Für die Oligomer- und Fibrillenpräparation wurden nicht-biotinyliertes Aß mit N-terminal biotinyliertem Aß in einem Verhältnis von 1:10 verwendet und das HFIP abgedampft (Concentrator 5301 von Eppendorf). Der entstandene Aß-Film wurde in einer finalen Konzentration von 80 µM in Natriumphosphatpuffer (10 mM, pH 7,4) aufgenommen und inkubiert (RT, 600 rpm). Die Inkubationszeit betrug bei der Oligomerpräparation 3 h und bei der Fibrillenpräparation 24 h. Zur Präparation von Monomeren wurde 100% N-terminal biotinyliertes Aß1-42 ohne Inkubation verwendet.

### Dichtegradientenzentrifugation

Die Dichtegradientenzentrifugation wurde im Anschluss an die Aß Präparation durchgeführt, um die jeweiligen Aß Spezies ihrer Größe entsprechend aufzureinigen. Dazu wurde ein Iodixanol-Gradient in 10 mM Natriumphosphatpuffer, pH 7.4, mit ansteigenden Konzentrationen von 50% bis 5% v/v lodixanol verwendet. 100 µl der Aß-Probe wurden aufgetragen und mittels Ultrazentrifugation aufgetrennt (3 h, 4 °C, 259.000 g). Im Anschluss wurde der Gradient in 14 Fraktionen zu je 140 µl fraktioniert. Monomeres Aß befinden sich in den ersten beiden oberen Fraktionen, Aß-Oligomere in den Fraktionen 4 bis 6 und Aß-Fibrillen in den Fraktionen 11 bis 13.

### Immobilisierung für SPR-Spektroskopie (Oberflächenplasmonresonanz)

Für SPR-Spektroskopie wurde ein T200 von Biacore (GE Healthcare) verwendet. Die mittels Dichtegradientenzentrifugation gereinigten Aß-Spezies wurden direkt mittels Biotin-Streptavidin-Kopplung auf einen Sensor Chip (Series S Sensor Chips SA) nach Herstellerangaben immobilisiert. Als Laufpuffer diente 1X PBS. Die Beladung erfolgte bei 25 °C und einer Flussgeschwindigkeit von 5 µl/min. Im Anschluss wurden die Flusszellen über Nacht bei einem stetigen Fluss von 30 µl/min von unspezifisch gebundenem Liganden befreit.

### Bindungskinetiken

Bindungskinetiken wurden ebenfalls mittels SPR-Spektroskopie an einem T200 Gerät von Biacore (GE Healthcare) gemessen. Die Standardbedingungen sind 25 °C und eine Flussgeschwindigkeit von 30 µl/min. Verschiedene Lyophilisate der D-Peptide wurden im Laufpuffer 1X PBS aufgenommen und seriell verdünnt. Als Methode diente eine "Single-Cycle"-Kinetik, wobei fünf ansteigende Analyt-Konzentrationen über die immobilisierten Flusszellen gepumpt werden. Die Kontaktzeiten wurden je nach Analyt 90-120 s für Assoziation und Dissoziation und 1800-5400 s für die finale Dissoziation gewählt. Die Sensorgramme wurden doppelt referenziert mit Hilfe einer nicht beladenen Flusszelle und den verwendeten Laufpuffer. Die Auswertung der Bindungskurven erfolgte mittels kinetischen Fit-Modellen (Heterogenes Bindemodell) unter Verwendung der Biacore T200 Evaluation Software (Version 2.0).

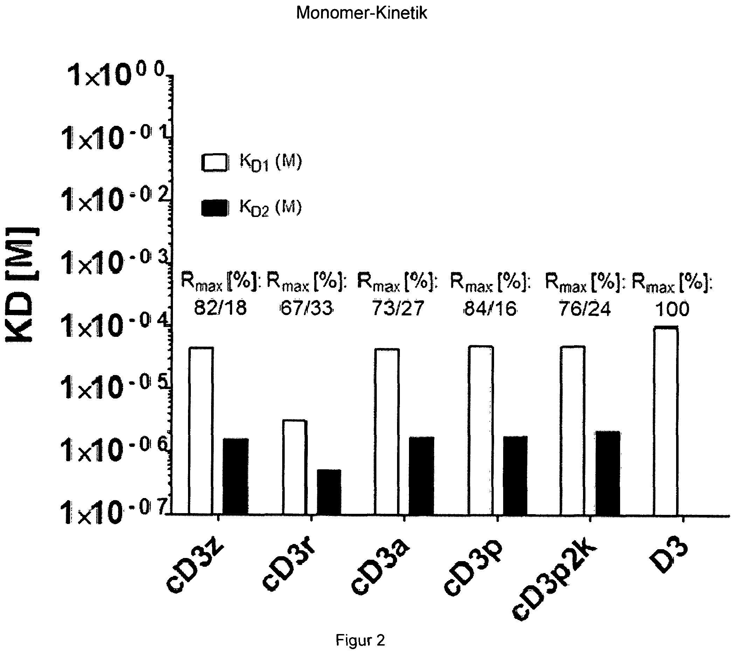

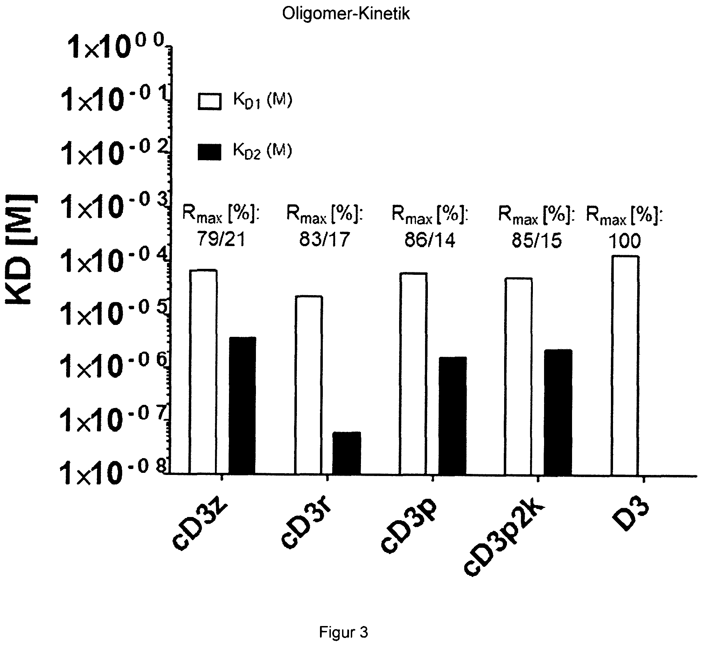

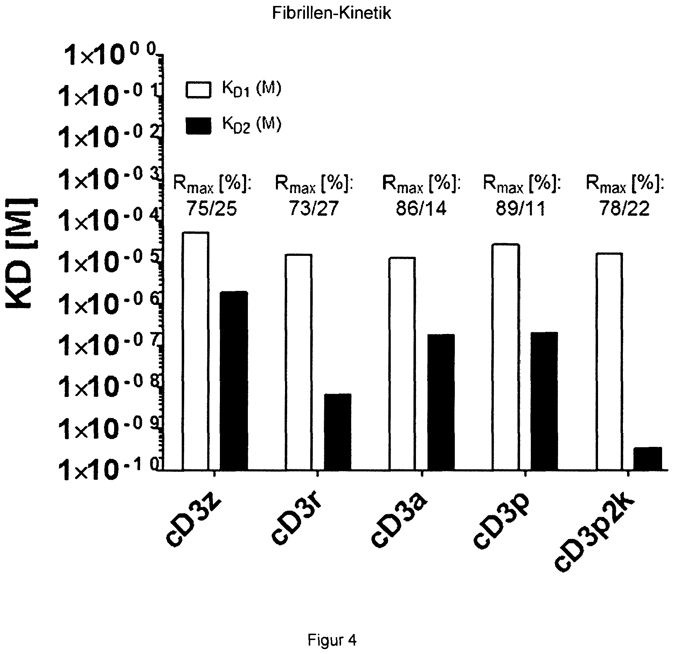

Die Figuren 2-4 zeigen die Ergebnisse zum Bindungsverhalten der offenbarungsgemäßen zyklisierten Peptide (Affinitätsstudie). In den Figuren sind Daten zur kinetischen Auswertungen der Bindungsstärke der verschiedenen Kandidaten an Amyloid-Beta-Monomere (Figur 2), Amyloid-Beta-Oligomere (Figur 3) und Amyloid-Beta-Fibrillen (Figur 4). Gezeigt sind jeweils die zwei Bindekonstanten, die beim Fitten eines heterogenen Bindemodells entstehen, und jeweils als weiße Balken und schwarze Balken aufgetragen sind. Wichtig ist, dass hier eine logarithmische Skala zu sehen ist, das bedeutet kleine Unterschiede in der Balkengröße sind große Unterschiede in der Dissoziationskonstanten K_{D}. Weiße Balken sind jeweils die low-afinitysites, also die niedrigaffinen Bindestellen und deren Bindungsstärke und die schwarzen Balken zeigen die hochaffinen Bindestellen und deren Bindungsstärke.

Es wird gezeigt, dass nur im Falle des linearen Peptids D3 ein homogenes 1:1-Bindemodell ausreicht, um die Bindungskurven zu fitten.

Es wird außerdem gezeigt, dass in fast allen Fällen bei den verwendeten zyklisierten Peptiden die weißen Balken zu den niedrigaffinen Bindestellen ähnlich hoch sind. Die großen Unterschiede entstehen vielmehr bei den hochaffinen Bindestellen, die als schwarze Balken dargestellt sind.

Ferner wird deutlich, dass bei den Monomeren das zyklische cD3r (SEQ ID NO: 5; nicht erfindungsgemäß) sehr gut abschneidet (Figur 2), also eine besonders hohe Affinität besitzt.

Bei den Oligomeren, die besonders interessant sind, bindet cD3r (nicht erfindungsgemäß) sogar um zwei Größenordnungen stärker als die anderen offenbarungsgemäßen zyklisierten Peptide (Figur 3).

Auch bei den Fibrillen schneidet das cD3r (nicht erfindungsgemäß) sehr gut im Vergleich zu den anderen offenbarungsgemäßen Peptiden ab, wobei hier als Besonderheit das erfindungsgemäße Peptid cD3P2K (SEQ ID NO: 11; nicht erfindungsgemäß) extrem gut abschneidet und bis in den sub-pM Bindebereich vorstößt (Figur 4).

Die Rₘₐₓ-Werte in den Figuren 2-4 geben an, wie stark der Beitrag des jeweiligen K_{D} zur Gesamtbeladungskapazität ist. Im Beispiel zu den Oligomeren (Figur 3) ist für das Peptid cD3z (nicht erfindungsgemäß) ein Rₘₐₓ von 79/21% angegeben. cD3z steht für cD3 zero, das heißt zyklisiertes D3 (nicht erfindungsgemäß) ohne weitere Aminosäureanhänge. Der weiße Balken zeigt an, dass die low affinity site insgesamt 79% der RU Gesamtbeladungsstärke ergeben kann und K_{D2} insgesamt 21% der RU. Das bedeutet, dass für dieses Peptid etwa ein Verhältnis von 1:4 an Bindestellen vorliegt, das heißt es gibt etwa 4 mal so viele low-affinitiy-sites wie high-affinity-sites.

Im Gegensatz hierzu lauten die K_{D1}-Werte und K_{D2}-Werte für das lineare D3 (nicht erfindungsgemäß) wie folgt:
Monomer: KD1=100, KD2=0
Oligomer: KD1=100, KD2=0
Fibrillen: nicht bestimmt

Die Tatsache, dass sich beim Fitten der experimentellen Bindungsdaten für das lineare Peptid D3 keine high affinity site ergibt, kann damit erklärt werden, dass es entweder nicht an die high affinity site bindet, oder aber dass die Affinitäten zu high affinity site und low affinity site nicht unterscheidbar sind.

Aus diesen Daten wird deutlich, dass das lineare D3 bei Amyloid-Beta-Oligomer nur eine low affinity site bindet, nicht aber eine high affinity site.

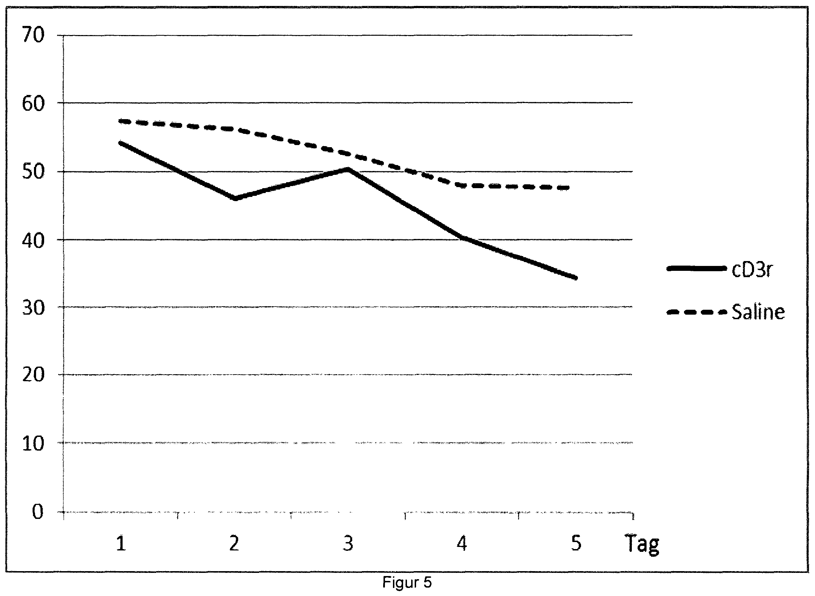

Figur 5 zeigt *in vivo* Daten aus dem MWW-Test. Mit dem Test wird das räumliche Gedächtnis von Tieren, hier von veränderten APPsDI-Mäusen gemessen. Diese Mäuse zeigen Verhaltensauffälligkeiten und biochemische Veränderungen, die einen Aspekt der Alzheimerschen Demenz, nämlich eine Störung des Gedächtnisses ausprägen.

Dabei wird die Zeit in Sekunden gemessen, die ein Tier benötigt, eine dicht unter der Wasseroberfläche versteckte Plattform in einem Wasserbottich wieder zu finden. Dies wird in mehreren Versuchen pro Tag an mehreren aufeinanderfolgenden Tagen gemessen. Eine statistische Auswertung ergibt dann, ob die behandelten Tiere im Vergleich zur Kontrollgruppe besser gelernt haben, die Plattform wiederzufinden. Aufgetragen ist die Suchzeit in Sekunden an fünf aufeinanderfolgenden Tagen für Mäuse die mit dem Peptid cD3r (nicht erfindungsgemäß) behandelt wurden im Vergleich zu Placebo-Experimenten (Saline).

Die mit dem zyklisierten Peptid cD3r (nicht erfindungsgemäß) behandelten Tiere haben signifikant gelernt (nicht parametrischer Friedmann Test, asymptotische Signifikanz cD3r =0,014; asymptotische Signifikanz Saline =0,238.

## Patentansprüche

1. Peptid, umfassend mindestens ein an eine Amyloid-Beta-Spezies bindendes Peptid, wobei das Peptid eine lineare Aminosäuresequenz aufweist, die es dazu befähigen, an A-Beta zu binden, und diese Eigenschaft verstärkt wird, in dem das Peptid durch kovalente Verknüpfung in zyklisierter Form vorliegt **gekennzeichnet durch** mindestens eine Struktur nach der Sequenz mit der SEQ ID NO:3 oder Homologe mit einer Identität von mindestens 83% zu SEQ ID NO:3 in zyklisierter Form.

2. Peptid nach Anspruch 1, **dadurch gekennzeichnet, dass** dieses im Wesentlichen aus D-enantiomeren Aminosäuren besteht.

3. Peptid nach einem der vorhergehenden Ansprüche, **dadurch gekennzeichnet, dass** das zyklisierte Peptid eine höhere Bindungsaffinität an Amyloid-Beta-Spezies hat als ein lineares Peptid, aus dem es sich ableiten lässt.

4. Peptid nach einem der vorhergehenden Ansprüche, **dadurch gekennzeichnet, dass** das zyklisierte Peptid eine höhere Effektivität bei der Verhinderung der Bildung und/oder der Endtoxifizierung und / oder der Eliminierung von Amyloid-Beta-Oligomer hat, als ein lineares Peptid, aus dem es sich ableiten lässt.

5. Peptid nach einem der vorhergehenden Ansprüche, **dadurch gekennzeichnet, dass** es Amyloid-Beta-Oligomere mit einer Dissoziationskonstante (KD-Wert) von höchstens 1 pM, bevorzugt 800, 600, 400, 200, 100, 10 nM, besonders bevorzugt 1.000, 900, 800, 700, 600, 500, 400, 300, 200, 100 pM, insbesondere bevorzugt höchstens 50 pM, am meisten bevorzugt höchstens 20 pM, bevorzugt sub-pM bindet.

6. Peptid nach einem der vorhergehenden Ansprüche, **gekennzeichnet durch** mindestens 2, 3, 4, 5, 6, 7, 8, 9, 10 oder mehr jeweils wirksam an A-Beta-Spezies bindende lineare Peptid-Bausteine, wobei das dadurch entstehende Gesamtpeptid kovalent verknüpft wurde und dadurch in zyklisierter Form vorliegt.

7. Peptid nach vorherigem Anspruch, **gekennzeichnet durch** die Verknüpfung identischer oder nicht-identischer Peptid-Bausteine.

8. Peptid nach einem der vorherigen zwei Ansprüche, **gekennzeichnet durch** Peptid-Bausteine, welche Kopf an Kopf, Schwanz an Schwanz oder Kopf an Schwanz linear gekoppelt sind.

9. Peptid nach einem der vorhergehenden Ansprüche, zur Verwendung in der Medizin.

10. Kit, enthaltend mindestens ein Peptid nach einem der Ansprüche 1 bis 9.

11. Sonde, enthaltend mindestens ein Peptid nach einem der Ansprüche 1 bis 9.

12. Zusammensetzung, enthaltend mindestens ein Peptid nach einem der Ansprüche 1 bis 9, zur Verwendung in der Behandlung von Morbus Alzheimer.

13. Verwendung des Peptids nach einem der Ansprüche 1 bis 9 als Sonde für in vivo-Imaging zur Identifizierung, qualitativen und/oder quantitativen Bestimmung von Amyloid-Beta-Spezies.

14. Peptid nach einem der Ansprüche 1 bis 9 zur Verwendung bei der Verhinderung der Bildung von Amyloid-Beta-Oligomeren und/oder deren Zerstörung und/oder deren Enttoxifizierung.

15. Peptid nach einem der Ansprüche 1 bis 9 zur Verwendung bei der Bildung von nicht toxischen Polymer-Amyloid-Beta-Oligomer-Komplexen.

## Claims

1. Peptide, comprising at least one amyloid-beta species binding peptide, the peptide having a linear amino acid sequence, which enables it to bind to A-beta, and this property is enhanced by the peptide being present in cyclized form by covalent linking, **characterized by** at least one structure according to sequence of SEQ ID NO:3 or homologues with an identity of at least 83% of SEQ ID NO:3 in cyclized form.

2. Peptide according to claim 1, **characterized in that** it consists essentially of D-enantiomeric amino acids.

3. Peptide according to any one of the preceding claims, **characterized in that** the cyclized peptide has a higher binding affinity to amyloid-beta species than a linear peptide, from which it can be derived.

4. Peptide according to any one of the preceding claims, **characterized in that** the cyclized peptide has a higher effectivity in the prevention of the formation and/or detoxification and/or elimination of amyloid-beta oligomer than a linear peptide from which it can be derived.

5. Peptide according to any one of the preceding claims, **characterized in that** it binds to amyloid-beta oligomers having a dissociation constant (K_{D} value) of at most 1 pM, preferably 800, 600, 400, 200, 100, 10 nM, more preferably 1,000, 900, 800, 700, 600, 500, 400, 300, 200, 100 pM, particularly preferably at most 50 pM, most preferably at most 20 pM, preferably sub-pM.

6. Peptide according to any one of the preceding claims, **characterized by** at least 2, 3, 4, 5, 6, 7, 8, 9, 10 or more linear peptide building blocks each binding effectively to A-beta species, the thus resulting total peptide having been covalently linked and therefore is pesent in cyclized form.

7. Peptide according to the preceding claim, **characterized by** the linking of identical or non-identical peptide building blocks.

8. Peptide according to any one of the two preceding claims, **characterized by** peptide building blocks which are linearly coupled head to head, tail to tail or head to tail.

9. Peptide according to any one of the preceding claims for use in medicine.

10. Kit containing at least one peptide according to any one of claims 1 to 9.

11. Probe containing at least one peptide according to any one of claims 1 to 9.

12. Composition containing at least one peptide according to any one of claims 1 to 9 for use in the treatment of Alzheimer's disease.

13. Use of the peptide according to any one of claims 1 to 9 as probe for the in vivo-imaging for the identification, qualitative and/or quantitative determination of amyloid-beta species.

14. Peptide according to any one of claims 1 to 9 for use in the prevention of the formation of amyloid-beta oligomers and/or their elimination and/or their detoxification.

15. Peptide according to any one of claims 1 to 9 for use in the formation of non toxic polymer-amyloid-beta-oligomer complexes.

## Revendications

1. Un peptide comprenant au moins un peptide se liant à une espèce bêta-amyloïde, le peptide ayant une séquence d'acide aminé linéaire que lui permet de se lier à l'A-Bêta, et cette propriété est renforcée par le peptide étant présent sous forme cyclisée par liaison covalente, **caractérisé par** au moins une structure selon la séquence avec SEQ ID NO: 3 ou homologues avec une identité d'au moins 83% à SEQ ID NO: 3 sous forme cyclisée.

2. Peptide selon la revendication 1, **caractérisé en ce qu'**il est essentiellement constitué d'acides aminés D-énantiomériques.

3. Peptide selon l'une des revendications précédentes, **caractérisé en ce que** le peptide cyclisé a une plus grande affinité de liaison aux espèces bêta-amyloïdes qu'un peptide linéaire, à partir duquel il peut être dérivé.

4. Peptide selon l'une des revendications précédentes, **caractérisé en ce que** le peptide cyclisé a une plus grande effectivité dans la prévention de la formation et/ou détoxification et/ou élimination de l'oligomère bêta-amyloïde qu 'un peptide linéaire à partir duquel il peut être dérivé.

5. Peptide selon l'une des revendications précédentes, **caractérisé en ce qu'**il lie oligomères bêta-amyloïdes avec une constante de dissociation (valeur KD) d'au maximum 1 pM, de préférence 800, 600, 400, 200, 100, 10 nM, plus préféré 1.000, 900, 800, 700, 600, 500, 400, 300, 200, 100 pM, particulairement préféré 50 pM au maximum, le plus favorisé de 20 pM au maximum, de préférence sub-pM.

6. Peptide selon l'une des revendications précédentes, **caractérisé par** au moins 2, 3, 4, 5, 6, 7, 8, 9, 10 ou plus des blocs de construction des peptides linéaires se liant chacun efficacement aux espèces bêta-amyloïdes, le peptide total résultant ayant été lié de manière covalente et donc est présent sous forme cyclisée.

7. Peptide selon la revendication précédente, **caractérisé par** la liaison des blocs de construction des peptides identiques ou non identiques.

8. Peptide selon l'une des deux revendications précédentes, **caractérisé par** des blocs de construction des peptides que sont couplés linéairement tête à tête, queue à queue ou tête à queue.

9. Peptide selon l'une des revendications précédentes pour l'utilisation en médecine.

10. Kit, comprenant au moins un peptide selon l'une des revendications 1-9.

11. Sonde, comprenant au moins un peptide selon l'une quelconque des revendications 1-9.

12. Composition, comprenant au moins un peptide selon l'une des revendications 1-9 pour l'application en le traitement de la maladie d'Alzheimer.

13. Utilisation du peptide selon l'une quelconque des revendications 1-9 comme sonde pour l'imagerie in vivo pour l'identification, détermination qualitative et/ou quantitative des espèces bêta-amyloïdes.

14. Peptide selon l'une des revendications 1-9 pour l'utilisation dans la prévention de la formation d'oligomères bêta-amyloïdes et/ou de leur élimination et/ou de leur détoxification.

15. Peptide selon l'une quelconque des revendications 1-9 pour l'utilisation dans la formation de complexes polymères-bêta-amyloïdes-oligomères non toxiques.
